(19) Europäisches Patentamt
European Patent Office
Office européen des brevets

(11) Veröffentlichungsnummer: **0 037 960**
**B1**

(12) # EUROPÄISCHE PATENTSCHRIFT

(45) Veröffentlichungstag der Patentschrift: **27.07.83**

(51) Int. Cl.$^3$: **C 07 C 25/10**, C 07 C 17/12

(21) Anmeldenummer: **81102447.0**

(22) Anmeldetag: **01.04.81**

(54) **Verfahren zur Herstellung von Trichlorbenzolen.**

(30) Priorität: **10.04.80 DE 3013888**

(43) Veröffentlichungstag der Anmeldung: **21.10.81 Patentblatt 81/42**

(45) Bekanntmachung des Hinweises auf die Patenterteilung: **27.07.83 Patentblatt 83/30**

(84) Benannte Vertragsstaaten: **DE FR GB**

(56) Entgegenhaltungen:
**DE-A-1 618 517**
**US-A-1 923 419**
**US-A-1 934 675**
***Ullmanns Encyklopädie der technischen Chemie* Bd. 9 (1975) p. 504—508**

(73) Patentinhaber: **BAYER AG, Zentralbereich Patente, Marken und Lizenzen, D-5090 Leverkusen 1, Bayerwerk (DE)**

(72) Erfinder: **Petruck, Gerd-Michael, Dr., Dörpfeldstrasse 49, D-4006 Erkrath 2 (DE)**
Erfinder: **Wambach, Raimund, Dr., Berta-von-Suttner-Strasse 65, D-5090 Leverkusen 1 (DE)**
Erfinder: **Wissner, Adolf, Dr., Am Wasserturm 13, D-5090 Leverkusen 3 (DE)**
Erfinder: **Hartung, Sigurd, Dr., Neue Kempener Strasse 246, D-5000 Köln 60 (DE)**

**Die Akte enthält technische Angaben, die nach dem Eingang der Anmeldung eingereicht wurden und die nicht in dieser Patentschrift enthalten sind.**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 037 960 B1

Verfahren zur Herstellung von Trichlorbenzolen

Die Erfindung betrifft ein Verfahren zur Herstellung von Trichlorbenzolen mit einem besonders hohen Gehalt an 1,2,3-Trichlorbenzol durch Chlorierung von o-Dichlorbenzol mit elementarem Chlor in Gegenwart eines Katalysators.

Trichlorbenzole im Rahmen der vorliegenden Erfindung sind isomere Trichlorbenzole, im wesentlichen des 1,2,3- und das 1,2,4-Trichlorbenzol.

Es ist bekannt, Trichlorbenzole durch Chlorierung von o-Dichlorbenzol in Gegenwart von feinverteiltem Eisen oder Eisen-(III)-chlorid herzustellen (BIOS Final Report 986, 404; Houben-Weyl Band V/3, 656 (1962); JA-PS 53 2329).

Es wurde ein Verfahren zur Herstellung von Trichlorbenzolen durch Chlorierung von o-Dichlorbenzol mit elementarem Chlor in Gegenwart eines Katalysators gefunden, das dadurch gekennzeichnet ist, daß ein Aluminiumchloridkatalysator, der 0,01 bis 0,1 Gew.-Teile Eisen bzw. Eisenchlorid, bezogen auf 100 Gew.-Teile Aluminiumchlorid, enthält, verwendet wird.

Das erfindungsgemäße Verfahren kann mit Hilfe der folgenden Reaktionsgleichung erläutert werden:

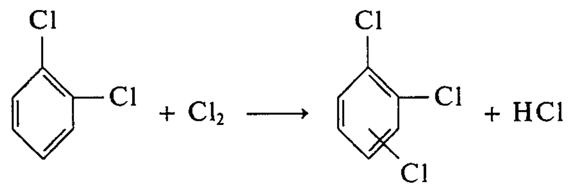

Als Aluminiumchlorid für das erfindungsgemäße Verfahren kann im allgemeinen wasserfreies handelsübliches Aluminiumchlorid verwendet werden. Geringe Feuchtigkeitsanteile des Aluminiumchlorids (bis zu etwa 2%) sind ohne Einfluß auf die katalytische Wirkung des Katalysators. Die Herstellung des Aluminiumchloridkatalysators ist an sich bekannt (Ullmanns Enzyklopädie der technischen Chemie, Bd. 7, 339 [1974]).

Der Katalysator kann beispielsweise durch Chlorieren von flüssigem Aluminium bei 750—800 °C in keramisch ausgekleideten, eisernen Kammern hergestellt werden.

Für das erfindungsgemäße Verfahren wird ein Aluminiumchlorid-Katalysator verwendet, der 0,01 bis 0,1 Gew.-Teile Eisen- bzw. Eisenchlorid, bezogen auf 100 Gew.-Teile Aluminiumchlorid, enthält. Eisen unter den erfindungsgemäßen Bedingungen liegt in der Regel als Chlorid vor.

Der Katalysator wird erfindungsgemäß in einer Menge von 0,1 bis 10,0 Gew.-Teilen, bevorzugt von 0,5 bis 1,0 Gew.-Teilen, bezogen auf 100 Gew.-Teile des o-Dichlorbenzols, eingesetzt.

Im allgemeinen wird das Chlor in nahezu äquivalenten Mengen, bezogen auf das o-Dichlorbenzol, eingeführt.

Das erfindungsgemäße Verfahren wird im allgemeinen im Temperaturbereich von 20 bis 120 °C, bevorzugt von 20 bis 50 °C, durchgeführt.

Das erfindungsgemäße Verfahren wird im allgemeinen bei einem Druck im Bereich von 0,1 bis 10 bar, vorzugsweise bei Atmosphärendruck, durchgeführt. Es wird auch bevorzugt, die Umsetzung bei autogenem Druck ineinem geschlossenen Gefäß durchzuführen.

Die Chlorierung erfolgt erfindungsgemäß durch Umsetzung von o-Dichlorbenzol mit elementarem Chlor. Der Reaktionsverlauf kann z. B. über die Dichte-Messung verfolgt werden.

Das erfindungsgemäße Verfahren kann sowohl diskontinuierlich als auch kontinuierlich durchgeführt werden.

Beispielsweise kann man das erfindungsgemäße Verfahren wie folgt durchführen:

In dem Reaktionsgefäß legt man das o-Dichlorbenzol und den Katalysator vor und leitet das Chlor ein, bis eine bestimmte Dichte erreicht ist. Nach Beendigung der Umsetzung wird das Chlorierungsprodukt beispielsweise mit Natriumcarbonat neutralisiert, mit Wasser gewaschen und im Vakuum destilliert. Man erhält ein Gemisch das im wesentlichen 1,2,3-Trichlorbenzol und 1,2,4-Trichlorbenzol enthält; beide Isomeren können in üblicher Weise durch fraktionierte Destillation getrennt werden.

In einer besonderen Ausführungsform des erfindungsgemäßen Verfahrens führt man die Umsetzung nur teilweise aus und erhält so die Trichlorbenzole mit großer Selektivität. Es ist bevorzugt, nur 20 bis 95%, insbesondere bevorzugt 60 bis 85% des eingesetzten o-Dichlorbenzols, umzusetzen.

Mit Hilfe des erfindungsgemäßen Verfahrens wird überraschenderweise die Ausbeute und die Selektivität an 1,2,3-Trichlorbenzol bei der Chlorierung von o-Dichlorbenzol gesteigert. Der Anteil an Nebenprodukten, im besonderen höherchlorierte Benzole, ist sehr gering.

Das nach dem erfindungsgemäßen Verfahren hergestellte Trichlorbenzol enthält einen besonders großen Anteil an 1,2,3-Trichlorbenzol, 1,2,3-Trichlorbenzol kann daraus besonders vorteilhaft isomerenfrei durch einfache physikalischen Trennmethoden erhalten werden.

Es ist jedoch auch möglich, das bei der Umsetzung anfallende Isomerengemisch direkt zu verwenden, beispielsweise als dielektrische Flüssigkeit (DE-OS 2 920 027).

## Beispiele 1 bis 5

In 735 g (5 Mol) o-Dichlorbenzol wird in Gegenwart von 6 g (0,81 Gew.-%) $AlCl_3$ bei 100°C so lange Chlor eingeleitet, bis eine Dichte von $D_4^{100} = 1{,}330$ g/cm³ erreicht ist. Das Rohprodukt wird mit einer wäßrigen $NaHCO_3$-Lösung neutral gewaschen, mit Wasser nachgewaschen und getrocknet.

Ausbeute: 792 g eines Gemisches aus

| | |
|---|---|
| 32,5% | o-Dichlorbenzol |
| 19,0% | 1,2,3-Trichlorbenzol |
| 38,2% | 1,2,4-Trichlorbenzol |
| 6,8% | 1,2,3,4-Tetrachlorbenzol |
| 3,2% | 1,2,4,5-Tetrachlorbenzol |
| 0,3% | Pentachlorbenzol |

Tabelle

| Bei-spiel Nr. | Einsatz o-DCB | Katalysator | | | Tempe-ratur | Umset-zungs-grad | Ausbeute Rohprod. | Zusammensetzung des Rohproduktes (Gew.-%) | | | | |
|---|---|---|---|---|---|---|---|---|---|---|---|---|
| | | Art | Menge | | | | | o-DCB | Trichlorbenzole | | Tetrachlorbenzole | |
| | [g] | | [g] | [Gew.-%] | [° C] | $\left[\frac{\text{Mol Chlor}}{\text{Mol Edukt}}\right]$ | [g] | | 1,2,3 | 1,2,4 | 1,2,3,4 | 1,2,4,5 |
| 1 | 735 | $AlCl_3$ | 6 | 0,81 | 100 | 0,650 | 792 | 32,5 | 19,0 | 38,2 | 6,8 | 3,2 |
| 2 | 735 | $AlCl_3$ | 6 | 0,81 | 20 | 0,678 | 814 | 29,0 | 21,1 | 43,2 | 5,0 | 1,7 |
| 3 | 735 | $AlCl_3$ | 3 | 0,4 | 50 | 0,692 | 825 | 29,5 | 20,2 | 41,3 | 5,8 | 2,3 |
| 4 | 735 | $AlCl_3$ | 6 | 0,81 | 50 | 0,650 | 811 | 32,4 | 19,4 | 39,7 | 6,0 | 2,4 |
| 5 | 735 | $AlCl_3$ | 6 | 0,81 | 50 | 0,838 | 827 | 17,9 | 20,9 | 46,7 | 10,0 | 4,2 |
| 6 | 1470 | $AlCl_3$ | 7,4 | 0,5 | 40 | 0,471 | 1628 | 49,5 | 15,9 | 31,1 | 2,4 | 0,9 |

Tabelle (Fortsetzung)

| Beispiel Nr. | Penta-Hexa-chlor-benzol | Selektivität | | | | Anteil der höherchlor. Benzole | | Ausbeute an 1,2,3-Tri-ClB |
|---|---|---|---|---|---|---|---|---|
| | | Anteil der Trichlorbenzole im Rohprod., davon | | | 1,2,3-Tri-ClB in % vom Umsatz | % vom Roh-produkt | % vom Umsatz | |
| | | [%] | 1,2,3-TCB | 1,2,4-TCB | | | | [g] |
| 1 | 0,3 | 57,2 | 33,2 | 66,8 | 28,1 | 10,3 | 15,3 | 150,5 |
| 2 | – | 64,3 | 32,8 | 67,2 | 29,7 | 6,7 | 9,4 | 171,7 |
| 3 | – | 62,0 | 32,6 | 67,4 | 28,7 | 8,4 | 11,9 | 166,6 |
| 4 | – | 59,1 | 32,8 | 67,2 | 28,7 | 8,5 | 12,6 | 157,3 |
| 5 | 0,3 | 67,6 | 30,9 | 69,1 | 25,5 | 14,5 | 17,7 | 172,8 |
| 6 | – | 47,0 | 33,8 | 66,2 | 30,5 | 3,3 | 6,3 | 258,9 |

Definitionen

1) Umsetzungsgrad $= \sum \left( \frac{G_i\,[\%] \cdot A\,[g] \cdot \Delta_{ni}}{MG_i\,[g] \cdot \text{Edukt}\,[\text{Mol}] \cdot 100} \right)_n \left[ \frac{\text{Mol Chlor}}{\text{Mol Edukt}} \right]$

$G_1$: Gehalt der Verbindung 1 im Rohprodukt (Gew.-%)
A: Gesamtausbeute (g)
$n_1$: Anzahl der Chloratome, die i mehr enthält als das Edukt (Mol)
$MG_1$: Molekulargewicht von 1

gibt also die Äquivalente Chlor an, die bei der Reaktion pro Mol Edukt eingeführt wurden.

2) Umsatz: gibt den Anteil an Edukt an, der chloriert wurde.

3) Ausbeute an 1,2,3-Trichlorbenzol: gibt an wieviel 1,2,3-Trichlorbenzol aus 5 Mol o-Dichlorbenzol erzeugt wurden.

**Patentansprüche**

1. Verfahren zur Herstellung von Trichlorbenzolen mit einem hohen Gehalt an 1,2,3-Trichlorbenzol durch Chlorierung von o-Dichlorbenzol mit elementarem Chlor in Gegenwart eines Katalysators, dadurch gekennzeichnet, daß ein Aluminiumchloridkatalysator, der 0,01 bis 0,1 Gew.-Teile Eisen bzw. Eisenchlorid, bezogen auf 100 Gew.-Teile Aluminiumchlorid, enthält, verwendet wird.

2. Verfahren nach Anspruch 1, dadurch gekennzeichnet, daß man den Katalysator in Mengen von 0,1 bis 10 Gew.-Teilen, bezogen auf 100 Gew.-Teile des o-Dichlorbenzols, einsetzt.

3. Verfahren nach den Ansprüchen 1 und 2, dadurch gekennzeichnet, daß man die Chlorierung im Temperaturbereich von 20 bis 120° C durchführt.

4. Verfahren nach den Ansprüchen 1 bis 3, dadurch gekennzeichnet, daß zur Chlorierung Chlor in einer etwa äquivalenten Menge zum o-Dichlorbenzol eingesetzt wird.

5. Verfahren nach den Ansprüchen 1 bis 4, dadurch gekennzeichnet, daß ein Druck von 0,1 bis 10 bar angewendet wird.

**Claims**

1. Process for the preparation of trichlorobenzenes having a high 1,2,3-trichlorobenzene content by chlorinating o-dichlorobenzene with elemental chlorine in the presence of a catalyst, characterised in that an aluminium chloride catalyst containing 0.01 to 0.1 part by weight of iron or iron chloride, relative to 100 parts by weight of aluminium chloride, is used.

2. Process according to Claim 1, characterised in that the catalyst is employed in amounts of 0.1 to 10 parts by weight, relative to 100 parts by weight of o-dichlorobenzene.

3. Process according to Claims 1 and 2, characterised in that the chlorination is carried out in the temperature range of 20 to 120° C.

4. Process according to Claims 1 to 3, characterised in that chlorine is employed in an amount approximately equivalent to that of o-dichlorobenzene for the chlorination.

5. Process according to Claims 1 to 4, characterised in that a pressure of 0.1 to 10 bar is used.

**Revendications**

1. Procédé de préparation de trichlorobenzènes à forte teneur en 1,2,3-trichlorobenzène par chloration de l'o-dichlorobenzène par le chlore élémentaire en présence d'un catalyseur, caractérisé en ce que l'on utilise un catalyseur à base de chlorure d'aluminium qui contient de 0,01 à 0,1 partie en poids de fer ou de chlorure de fer pour 100 parties en poids de chlorure d'aluminium.

2. Procédé selon la revendication 1, caractérisé en ce que le catalyseur est mis en oeuvre en quantités de 0,1 à 10 parties en poids pour 100 parties en poids de l'o-dichlorobenzène.

3. Procédé selon les revendications 1 et 2, caractérisé en ce que l'on effectue la chloration dans l'intervalle de température de 20 à 120° C.

4. Procédé selon les revendications 1 à 3, caractérisé en ce que, à la chloration, on utilise le chlore en quantité à peu près équivalente à celle de l'o-dichlorobenzène.

5. Procédé selon les revendications 1 à 4, caractérisé en ce que l'on opère sous une pression de 0,1 à 10 bars.